# EUROPEAN PATENT SPECIFICATION

(11) **EP 2 692 279 B1**
(45) Date of publication and mention of the grant of the patent: **30.08.2017**
(21) Application number: 12765424.2
(22) Date of filing: 23.03.2012
(51) Int. Cl.: A61B 1/04, A61B 1/00

(54) **ENDOSCOPE DEVICE**
ENDOSKOPVORRICHTUNG
DISPOSITIF D'ENDOSCOPE

(30) Priority: 29.03.2011 JP 2011072412
(43) Date of publication of application: 05.02.2014
(73) Proprietor: Panasonic Corporation, Kadoma-shi, Osaka 571-8501 (JP)
(72) Inventor: KOHNO, Haruhiko, Osaka-shi, Osaka 540-6207 (JP)
(74) Representative: Eisenführ Speiser
(86) International application number: PCT/JP2012/002045
(87) International publication number: WO 2012/132372

(56) References cited:
- EP-A1- 1 284 120
- EP-A1- 2 123 210
- EP-A1- 2 294 967
- WO-A1-2012/046413
- JP-A- 8 322 787
- JP-A- 11 253 390
- JP-A- 2004 180 781
- JP-A- 2005 312 555
- JP-A- 2005 334 237
- JP-A- 2007 075 604
- JP-A- 2007 195 799
- JP-A- 2008 502 384
- US-A1- 2007 270 636
- US-B1- 6 221 007

## Description

### FIELD OF THE INVENTION

The present invention relates to an endoscope device capturing an image of an inside of an object that cannot be observed directly from the outside, particularly to an endoscope device to be used with a treatment tool for accessing the object.

### BACKGROUND ART

A disposable casing for coupling an endoscope to a tool for a single use according to EP 1 284 120 A1 comprises at least one endoscope channel receiving the endoscope. The endoscope channel is designed as an endoscope/working channel for additionally receiving the tool and has an oval inner cross-section and/or at least one, preferably two guide webs for guiding the endoscope and the tool. The endoscope receives only the endoscope and has at least one working channel separate from the endoscope channel for receiving the tool. The endoscope channel and/or working channel has a circular inner cross-section.

An endoscopic instrument according to US 2007/270636 A1, particularly useful for the non-invasive treatment of some digestive system pathologies, has a flexible and elongate main body that houses a vision device for taking images of an organ internal area, comprising a first working arm for the use of tools, and further comprises a second working arm for the use of tools apt to be operated independently with respect to said first working arm, allowing an endoscopic treatment of a wide range of pathologies that at present are treatable only surgically.

An endoscopic instrument according to EP 2 294 967 A1 having an operating part arranged at a proximal end of a shaft and an instrument head arranged at a distal end of the shaft, where the operating part is connected to the instrument head by the shaft. Multiple instrument channels are guided through the shaft, and an opening is formed on the instrument head. The instrument channels are operated obliquely and outwardly along a distal direction with respect to a central axis of the instrument head in a section of the instrument channels that adjoin distal openings, where the openings are formed in the channels.

US 6,221,007 B1 discloses a device according to the preamble of claim 1.

In some conventional rigid endoscopes having a highly rigid insertion portion, a solid-state image sensing device is mounted at a distal end of the insertion portion, a signal line and a light guide (fiber) for the solid-state image sensing device are passed through a small diameter portion extending to a rear end of the insertion portion, and the insertion portion and a hollow tubular portion that receives a treatment tool, such as a catheter, a forceps or the like, are received in a sheath (refer to Patent Document 1).

### PRIOR ART DOCUMENT (S)

### PATENT DOCUMENT (S)

[Patent Document 1] JP2615048B

### SUMMARY OF THE INVENTION

### TASK TO BE ACCOMPLISHED BY THE INVENTION

In contrast to a case where the treatment tool is inserted through a hole separate from the hole through which the endoscope is inserted, for example, the endoscope disclosed in Patent Document 1 enables the endoscope and the treatment tool to be inserted into a single hole, and thus, makes it easier to close the hole after surgery.

However, in the endoscope disclosed in Patent Document 1, the image sensing device is fixed, and image capture is possible only in a direction fixed obliquely relative to the axial direction of the insertion portion. Therefore, to perform image capture in a range of 360 degrees about the axis of the insertion portion, it is necessary to rotate the sheath around the axis. However, rotation of the sheath for each image capture may change the shape of the hole, and is also cumbersome to be performed.

The present invention is made to address the aforementioned problem, and a primary object of the present invention is to provide an endoscope device which, when operation is performed in a state where the endoscope and a treatment tool are inserted into the same hole, can prevent a change in the shape of the hole and ensure excellent operability.

### MEANS TO ACCOMPLISH THE TASK

To accomplish the above task, the present invention provides an endoscope device, as defined in claim 1

### EFFECT OF THE INVENTION

Thus, according to the present invention, the guide portion guiding the treatment tool is formed integrally in the insertion member that movably supports the imaging unit at the distal end thereof, whereby mere insertion of the endoscope device through a single hole enables capturing of an image of any part within a predetermined range without need for rotation of the insertion member, while preventing a change in the shape of the hole and facilitating access of the treatment tool to a target part.

### BRIEF DESCRIPTION OF THE DRAWINGS

FIG. 1 is a an overall perspective view of an endoscope device according to the present invention;
FIG. 2 is a cross-sectional view taken along line II-II in FIG. 1;
FIG. 3 is a cross-sectional view of the endoscope device according to the present invention;
FIG. 4 is a perspective view of a main portion of an inside of an insertion portion;
FIG. 5 is a diagram showing an image capturing range and an accessing state of a treatment tool;
FIG. 6A is a diagram showing a state where the treatment tool has not been mounted in another embodiment of the present invention, and FIG. 6B is a diagram showing a state where the treatment tool has been mounted in the embodiment;
FIG. 7 is a diagram showing an exemplary state of use of the endoscope device; and
FIG. 8A is a diagram similar to FIG. 7 and showing another exemplary state of use of the endoscope device, and FIG. 8B is a diagram similar to FIG. 8A but with the endoscope being removed.

### EMBODIMENTS FOR CARRYING OUT THE INVENTION

A first aspect of the present invention provides an endoscope device, including: an imaging unit capturing an image of an object; and an insertion member movably supporting the imaging unit at a distal end thereof, wherein the insertion member includes: a tubular portion that receives an operation member coaxially with the imaging unit, the operation member being configured to change an image capturing direction of the imaging unit; and a guide portion formed integrally with the tubular portion to guide a treatment tool for accessing the object such that the treatment tool is displaceable in a longitudinal direction of the tubular portion on a lateral side of the tubular portion.

According to the first aspect, the guide portion guiding the treatment tool is formed integrally in the insertion member that movably supports the imaging unit at the distal end thereof, whereby mere insertion of the endoscope device through a single hole enables capturing of an image of any part within a predetermined range without need for rotation of the insertion member, while preventing a change in the shape of the hole and facilitating access of the treatment tool to a target part.

A second aspect of the present invention provides an endoscope device, including: an imaging unit capturing an image of an object; an imaging holder holding the imaging unit; two systems of driving force transmission mechanisms each including a pair of driving rods having distal ends connected to the imaging holder at mutually diagonal positions; a driving apparatus disposed on a base end side of the driving rods and driving forward and backward at least one of the driving rods in each of the driving force transmission mechanisms to displace the imaging holder; and an insertion member extending from a base member of the driving apparatus, wherein the insertion member integrally includes: a tubular portion that receives the imaging unit, the imaging holder and the driving force transmission mechanisms; and a guide portion that guides a treatment tool for accessing the object such that the treatment tool is displaceable in a longitudinal direction of the tubular portion on a lateral side of the tubular portion.

According to the second aspect, an insertion member extends from the base member of the driving apparatus for displacing the imaging holder, the insertion member includes a tubular portion and a guide portion provided on the lateral side of the tubular portion, the tubular portion receives the imaging unit, the imaging holder and the driving force transmission mechanisms, and the guide portion guides the treatment tool so as to be displaceable, and therefore, it is possible to easily capture an image of an object by displacing (e.g., pan/tilt) the imaging unit, and make the insertion member approach the object easily and quickly. Further, since the imaging unit and the guide portion for guiding the treatment tool are provided integrally in the insertion member, the direction of displacement of the treatment tool can be within the image capturing range, and this facilitates access of the treatment tool to the object.

In a third aspect of the present invention, the guide portion is formed of a groove having an opening of a size determined so as to be capable of preventing the treatment tool from coming off from the groove sideways. According to the third aspect, it is possible to remove the wall thickness on the lateral side of the insertion member and thereby reduce the width of the insertion member, and this results in a smaller hole cut in the patient's body for insertion of the insertion member. Further, the structure allows the treatment tool or the like to protrude to outside through the side opening, and this increases the number of available functions.

In a fourth aspect of the present invention, the guide portion includes a pair of guide portions radially interposing the tubular portion therebetween. According to the fourth aspect, multiple treatment tools may be mounted in accordance with an operation to be performed. For example, treatment tools for different operations may be combined, and thus, versatility is improved.

In a fifth aspect of the present invention, a part of the insertion member where the guide portion is provided has an elliptical cross section. According to the fifth aspect, by inserting the insertion member such that the major axis of the insertion member is aligned with the direction of cutting, widening of the hole fonned by the cutting can be suppressed.

Embodiments of the present invention will be described below with reference to the drawings.

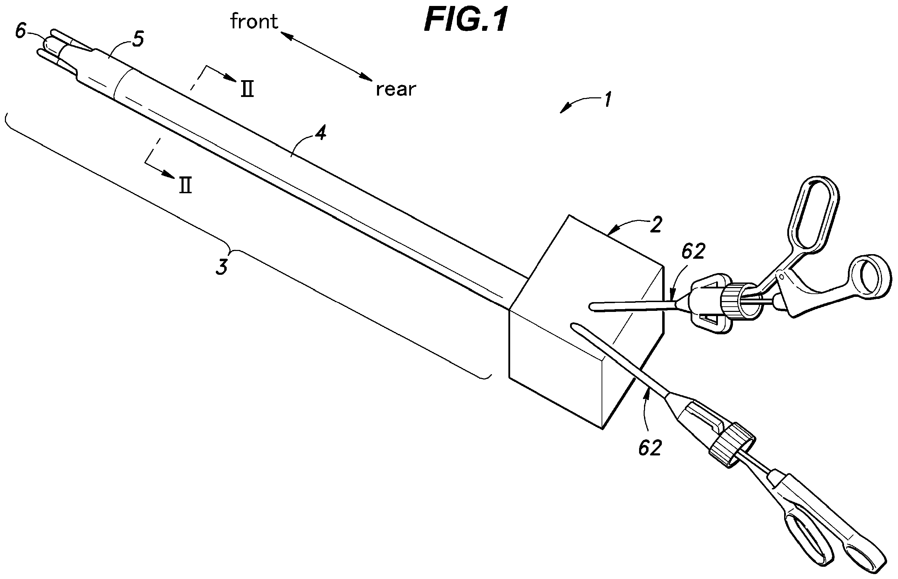

FIG. 1 is an overall perspective view showing an endoscope device 1 according to an embodiment of the present invention. As a rigid endoscope for medical or industrial use, the illustrated endoscope device 1 primarily includes a main body 2 and an insertion portion 3 extending forward from the main body 2 and serving as an insertion member. The insertion portion 3 has a small diameter and high rigidity so as not to be bent easily. The insertion portion 3 is inserted into an object (not shown in the drawing, e.g., patient's body). The insertion portion 3 is configured such that a treatment tool (forceps 62, for example) can pass through the inside of the insertion portion 3.

As shown in FIG. 1, a proximal end (base end) part of the insertion portion 3 is formed of a protection rod 4 fixed to the main body 2, and an intermediate cover 5 made of resin or glass is connected to a front end (distal end) of the protection rod 4. The intermediate cover 5 is formed to have a substantially elliptical cross section at the part connected with the protection rod 4 and to have a circular cross section at the front end thereof, the diameter of the circular cross section being the same as the length of the minor axis of the substantially elliptical cross section. Attached to the front end of the intermediate cover 5 is a distal end cover 6 that is made of glass or transparent resin such as acrylic or polycarbonate, etc., to serve as an imaging window that is transmissive to light. The part of the distal end cover 6 that is fitted over the front end part of the intermediate cover 5 is formed to have a cylindrical shape, and the tip end of the distal end cover 6 is formed to have a semispherical shape.

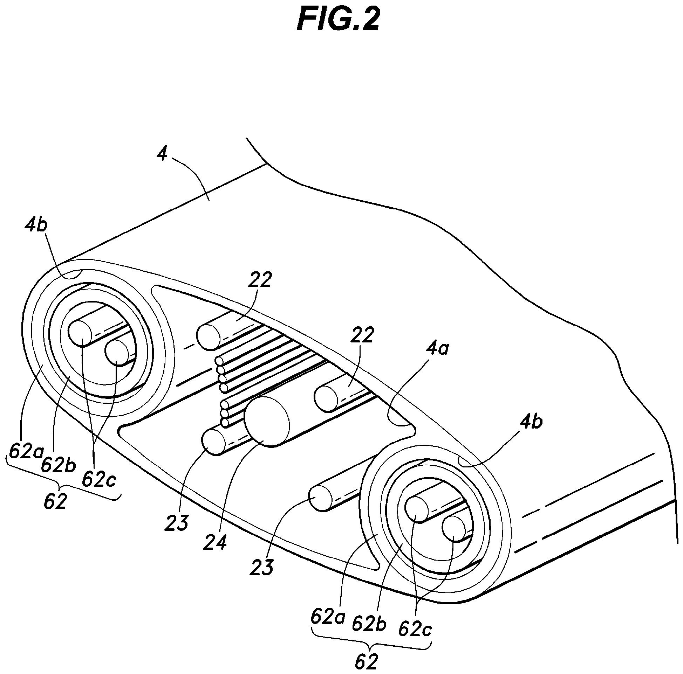

FIG. 2 is a cross-sectional view of the insertion portion 3 taken along line II-II in FIG. 1. The protection rod 4 as a whole has a substantially elliptical cross section, and includes a tubular portion 4a disposed at a central portion thereof and guide portions 4b formed of a pair of through holes disposed on either lateral side the tubular portion 4a. In FIG. 2, a treatment tool (e.g., a forceps 62) extends inside each guide portion 4b.

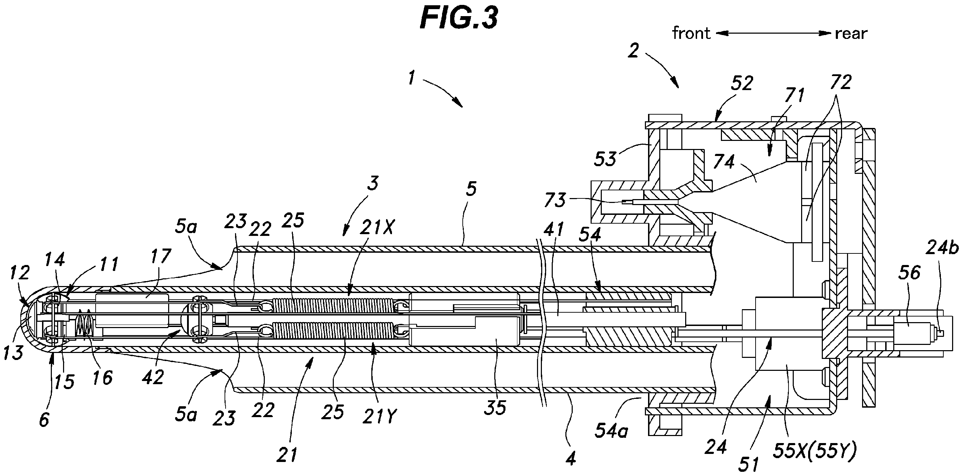

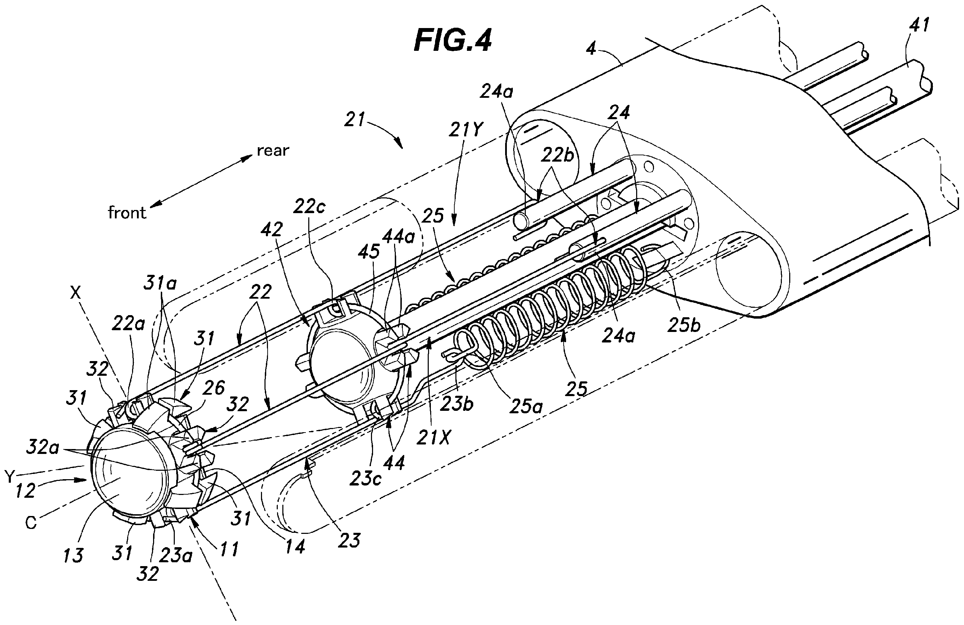

FIG. 3 is a horizontal cross-sectional view of the endoscope device 1, and FIG. 4 is a perspective view of a main portion of an inside of the insertion portion 3. As described in the foregoing, the endoscope device 1 according to the present invention is configured to include the protection rod 4 having the tubular portion 4a and the pair of guide portions 4b on either side of the tubular portion 4a. Cables for the imaging system and a linking mechanism serving as an operating member for the imaging system are passed through the tubular portion 4a, while a forceps, a catheter or the like is passed through either guide portion 4b.

First, description will be given of the imaging system in this endoscope device 1 with reference to FIGS. 3 and 4. An imaging unit 12 is disposed in the distal end of the internal space of the insertion portion 3, where the imaging unit 12 is held by an imaging holder 11 so as to be rotatable around two axes (X axis and Y axis in FIG. 4), whereby the imaging unit 12 may capture an image of an object while changing a viewing direction. The imaging unit 12 has an objective lens system 13 and a solid-state image sensing device 14, the objective lens system 13 being composed of one or more optical lenses, and the solid-state image sensing device 14 being disposed in the rear of the objective lens system 13 such that an image of light from the lenses is formed on a light receiving surface. In this embodiment, the imaging unit 12 has a viewing angle of approximately 170 degrees. In the description below, when components associated with rotation around the two axes (X axis and Y axis) of the imaging unit 12 are to be distinguished from each other, they will be referred to by a component name or a reference numeral with a suffix (X or Y). It is also to be noted that the main scanning direction and sub-scanning direction of the solid-state image sensing device 14 correspond to the X axis and Y axis, respectively.

The imaging holder 11 has a cylindrical main body into which the rear portion of the objective lens system 13 is fitted. The solid-state image sensing device 14 is attached to the rear side of the main body of the imaging holder 11. The solid-state image sensing device 14 may be any known image sensor such as that composed of a CMOS (Complementary Metal Oxide Semiconductor), for example.

A rear side of the solid-state image sensing device 14 is bonded to and directly supported by a joint 15 of a flexible cable 16 through a BGA (Ball Grid Array) connection. The joint 15 is connected to a drive board 17 (refer to FIG. 3) by the flat flexible cable 16 for transmitting and receiving a variety of signals and for supplying electric power. A circuit for converting voltage of the power source to drive the solid-state image sensing device 14, a clock generation circuit, etc. are provided on the drive board 17. An AD converter may be mounted on the drive board 17 in a case where the solid-state image sensing device 14 does not include a built-in AD converter. The drive board 17 is positioned between the imaging unit 12 and a later-described relay holder 42 in a front-back direction, and is fixed to the inner peripheral surface of the intermediate cover 5. A cable (not shown in the drawings) that transmits and receives captured image data and the like is provided between the drive board 17 and an image processor (not shown in the drawings) on the main body 2 side.

Further, two systems of driving force transmission mechanisms 21X and 21Y for rotating the imaging unit 12 in two axial directions are provided in the internal space of the insertion portion 3. The two systems of driving force transmission mechanisms 21X and 21Y have structures similar to each other. Each of the driving force transmission mechanisms 21X and 21Y includes a pair of a first driving rod 22 and a second driving rod 23 having distal ends connected to the imaging holder 11 at mutually diagonal positions and extending in the front-back direction, the first and second driving rods 22 and 23 serving as an operation member of the imaging unit 12.

As shown in FIG. 4, the first and second driving rods 22 and 23 each consist of a flexible rod-shaped member composed of a so-called spring steel, and respectively have distal end portions 22a and 23a, each of which is wound once and formed into a substantially annular shape (spiral shape). A ring member 26, which also is composed of a spring steel and is attached to an outer periphery of the main body of the imaging holder 11, is passed through the openings of the distal end portions 22a and 23a. The ring member 26 is loosely held in the openings of the distal end portions 22a and 23a.

The outer periphery of the main body of the imaging holder 11 is provided with four ribs 31 spaced apart from each other at equal distances in the circumferential direction. Furthermore, four support guides 32 are provided between the ribs 31 to position the distal end portions 22a and 23a of the first and second driving rods 22 and 23, respectively. A groove extending in the circumferential direction is provided in an intermediate portion in the front-back direction of each of the ribs 31, and the ring member 26 is fitted into the grooves. The ring member 26 is composed of a circular metal ring having one cut portion. When inserted into the openings of the distal end portions 22a and 23a of the first and second driving rods 22 and 23, the ring member 26 is temporarily deformed to open the cut portion, thereby enabling the insertion.

Each of the support guides 32 includes a pair of guide pieces 32a opposed to each other in the circumferential direction. Similarly to the ribs 31, a groove is formed in an intermediate portion in the front-back direction of each of the guide pieces 32a, and the ring member 26 is fitted in these grooves. Each of the distal end portions 22a and 23a of the first and second driving rods 22 and 23 is disposed in a space defined between a corresponding pair of the guide pieces 32a. Thus, the circumferential movement of the distal end portions 22a and 23a is limited within a predetermined range.

When assembled, the cut portion of the ring member 26 is opened in advance, the openings of the distal end portions 22a and 23a (two each) are passed through the ring member 26, the distal end portions 22a and 23a are positioned with an appropriate jig, and the ring member 26 engaged with the distal end portions 22a and 22d is moved in the front-back direction along an optical axis (center axis C of the insertion portion 3) so as to be fitted to the imaging holder 11. Thereby, the ring member 26 is fitted into the grooves of the ribs 31 and the support guides 32, while the distal end portions 22a and 23a are received between the guide pieces 32a of the respective support guides 32 with some play relative to the ring member 26. Thus, the imaging holder 11 is engaged so as to be displaceable according to the forward and backward movement of the first and second driving rods 22 and 23.

In another method of engaging the imaging holder 11 with the distal end portions 22a and 23a of the first and second driving rods 22 and 23, the ring member 26 may be fitted into the grooves of the ribs 31 and the support guides 32 in advance. Then, the ring member 26 is rotated in the circumferential direction of the imaging holder 11 with the cut portion slightly opened, and the distal end portions 22a and 23a is each inserted at the position of the corresponding guide pieces 32a when the open portion of the ring member 26 reaches the position of the guide pieces 32a. In this case, it is preferred that the ring member 26 be configured such that the cut portion is opened in a non-load state and that the distance between opposing ends of the ring member 26 at the open portion be equal to or greater than the diameter of the first and second driving rods 22 and 23.

In each driving force transmission mechanism 21, a hook-shaped proximal end portion 22b of the first driving rod 22 is connected with a distal end portion 24a of a metallic motor connection rod 24 extending in the front-back direction, as shown in FIG. 4. The motor connection rod 24 is movably passed through a guide hole in a cylindrical guide member 35 (see FIG. 3) that is fixedly supported by a support shaft 41, and a proximal end portion 24b of the motor connection rod 24 extends to a rear portion of the main body 2, as shown in FIG. 3. Further, a hook-shaped proximal end portion 23b of the second driving rod 23A is connected to a distal end portion 25a of a tension spring (elastic member) 25 extending in the front-back direction. A proximal end portion 25b of the tension spring 25 is fixed to a front surface of the guide member 35 (see FIG. 3).

The support shaft 41 has a base end fixed to the main body 2 and extends from the main body 2 along the center axis of the insertion portion 3; namely, the support shaft 41 extends in the front-back direction of the insertion portion 3. A hemispherical relay holder 42 is attached to a distal end of the support shaft 41 to support the first and second driving rods 22 and 23. The guide member 35 (see FIG. 3) is fixed to an intermediate portion of the support shaft 41.

A spherical ball member (not shown in the drawings) is attached to a distal end portion 41a of the support shaft 41. The ball member is slidably received in a receiver (not shown in the drawings) having a spherical sliding surface provided in a rear portion or inside of the relay holder 42, thereby forming a ball joint. The relay holder 42 is tiltably held at the distal end of the support shaft 41 through the ball joint. The maximum outer diameter of the relay holder 42 is set smaller than the external casing (intermediate cover 5 herein) of the insertion portion 3, so as to prevent the relay holder 42 from coming into contact with the external casing of the insertion portion 3 when the relay holder 42 is tilted.

The first and second driving rods 22 and 23 have intermediate portions 22c and 23c, respectively, which are each wound once and formed into a substantially annular shape, similarly to the aforementioned distal end portions 22a and 23a. A ring member 45, which is attached to an outer periphery of the main body of the relay holder 42, is passed through the openings of the intermediate portions 22c and 23c. Similarly to the ring member 26, the ring member 45 is a circular ring composed of a spring steel and having one cut portion, and is loosely held in the openings of the intermediate portions 22c and 23c.

Four guides 44 are provided on the outer periphery of the main body of the relay holder 42, the four guides 44 having structures similar to those of the support guides 32 of the imaging holder 11 described above. Each of the guides 44 is composed of a pair of guide pieces 44a for positioning the corresponding one of the intermediate portions 22c and 23c of the first and second driving rods 22 and 23. Each of the guide pieces 44a is provided with a groove, into which the ring member 45 is fitted. The relay holder 42 may be provided with ribs similar to the ribs 31 of the imaging holder 11.

Similarly to the case of the imaging holder 11, when assembled, the cut portion of the ring member 45 is opened in advance, and then, the openings of the intermediate portions 22c and 23c (two each) are passed through the ring member 45 and are positioned with an appropriate jig. Subsequently, the ring member 45 is moved in the front-back direction along the center axis C so as to be fitted to the relay holder 42. Thereby, the ring member 45 is fitted into the grooves of the guides 44, while the intermediate portions 22c and 23c are received between the guide pieces 44a of the respective guides 44 with some play relative to the ring member 45. Thus, the relay holder 42 is engaged so as to be tiltable according to the forward and backward movement of the first and second driving rods 22 and 23.

It is to be noted that the aforementioned "another method" for engaging the imaging holder 11 with the distal end portions 22a and 23a of the first and second driving rods 22 and 23 may be used as it is as a method for engaging the relay holder 42 with the intermediate portions 22c and 23c of the first and second driving rods 22 and 23.

The configuration connecting the intermediate portions 22c and 23c of the first and second driving rods 22 and 23 with the relay holder 42 allows the driving rods 22 and 23 to function as a linking mechanism that links the relay holder 42 and the imaging holder 11, thus achieving stable rotation of the imaging unit 12 around the two axes (X and Y axes in FIG. 4) in a small space.

In rotation of the imaging unit 12 by the driving force transmission mechanism 21X, the rotation axis (X axis in FIG. 4) substantially coincides with an axis passing through the distal end portions 22a and 23a of the first and second driving rods 22 and 23 paired in the driving force transmission mechanism 21Y (the other system). Similarly, in rotation of the imaging unit 12 by the driving force transmission mechanism 21Y, the rotation axis (Y axis in FIG. 4) substantially coincides with an axis passing through the distal end portions 22a and 23a of the first and second driving rods 22 and 23 paired in the driving force transmission mechanism 21X. Thus, the X axis and Y axis are not fixed to the positional relationship shown in FIG. 4, but are displaced during rotation of the imaging unit 12 (i.e., according to the displacement or deformation of the driving rods 22 and 23). The X axis and the Y axis may be orthogonal to each other so that a pan/tilt function is readily available in image capturing. The two axes, however, do not have to be orthogonal to each other, and they may simply cross each other without being adjusted to be orthogonal to each other, or may be in a skewed relationship with each other depending on the situation.

The main body 2 of the endoscope 1 shown in FIG. 3 includes a driving apparatus 51 that drives forward and backward the first and second driving rods 22 and 23 in the driving force transmission mechanisms 21. A base member 53 forming a front part of a case 52 of the driving apparatus 51 is connected with a flange 54a of a fixing member 54 to which the proximal end of the insertion portion 3 (protection rod 4) is attached. Further, two electric motors 55X and 55Y are provided in the case 52 to generate driving force for the driving force transmission mechanisms 21X and 21Y, respectively. It is to be noted that the electric motor 55Y is not shown in FIG. 3 because the electric motor 55Y is disposed behind the electric motor 55X in FIG. 3.

The electric motors 55X and 55Y are direct acting stepping motors each having a motor shaft (screw shaft; not shown in the drawings) to convert rotation movement into linear movement. The stepping motors are driven by what is generally-called a micro-step drive. The proximal end portions 24b of the motor connection rods 24 in the respective driving force transmission mechanisms 21 are disposed adjacent to the motor shafts of the electric motors 55X and 55Y extending in the front-back direction and are connected to the motor shafts through connecting members 56. With such configuration, the first driving rod 22 and the second driving rod 23 in the respective driving force transmission mechanisms 21 can move forward and backward along the axis (front-back direction) of the insertion portion 3 in a substantially linear way according to the rotation amount of the electric motors 55X and 55Y (i.e., amount of movement of the motor shafts).

The driving apparatus 51 is further provided with two home position sensors (not shown in the drawings) that detect the home positions of the motor shafts (i.e., motor connection rods 24) of the electric motors 55X and 55Y, respectively. Each home position sensor includes a PI (photointerrupter) sensor having a light emitter and a light receiver opposed to each other. Each of the connecting members 56 is provided with a shutter piece (not shown in the drawings) projecting therefrom to block the light emitted from the light emitter of the corresponding home position sensor. With this configuration, when the motor shaft to which the connecting member 56 is attached is moved, the position at which the light from the light emitter is completely blocked as a result of insertion of the shutter piece between the light emitter and the light receiver can be recognized as the home position of the motor shaft.

The main body 2 shown in FIG. 3 further includes an illumination apparatus 71 for image capturing. The illumination apparatus 71 has a plurality of LEDs (light emitting diodes) 72 and a transparent resin light guide 74 disposed in front of the LEDs 72 and guiding the light output from the LEDs 72 to four optical fiber cables 73. In accordance with the purpose of image capturing, the LEDs 72 can selectively output white light, ultraviolet light or infrared light or can output the lights simultaneously. The optical fiber cables 73 extend to the vicinity of the imaging unit 12 through the inside of the insertion portion 3, and are thus capable of irradiating light onto an object from distal ends of the cables.

The viewing direction of the imaging unit 12 in the present invention is not fixed, and image capture is possible in any direction in 360 degrees around the center axis C (refer to FIG. 4). In the initial state, the viewing direction of the imaging unit 12 is directed forward along the center axis C of the insertion portion. In this state, a backward force by the tension spring 25 (urging force of the spring) is exerted on the proximal end portion 23b of the second driving rod 23, creating a predetermined tensile force between the proximal end portion 23b and the intermediate portion 23c of the second driving rod 23 supported by the ring member 45 of the relay holder 42. The tensile force exerted by the tension spring 25 is transmitted through the relay holder 42 to the intermediate portion 22c of the first driving rod 22 supported by the ring member 45 of the relay holder 42, also creating a tensile force between the intermediate portion 22c and the proximal end portion 22b of the first driving rod 22.

To change the viewing direction of the endoscope 1, the electric motor 55X shown in FIG. 3 is activated to retract the motor connection rod 24, for example. This causes the imaging unit 12 to rotate around the X axis to change the viewing direction. The outer peripheral surfaces 31a of the ribs 31 of the imaging holder 11 each have a curvature identical with that of the inner peripheral surface of the semispherical distal end projection of the distal end cover 6, such that, in rotation of the imaging unit 12, the outer peripheral surfaces 31a of the ribs 31 are in slidable contact with the inner peripheral surface of the distal end projection to guide the rotation. The tilt angle of the center axis of the imaging unit 12 relative to the center axis C of the insertion portion may be set at any angle in the range from 0 to 30 degrees, for example.

At this time, a backward force (driving force of the electric motor 55X) is exerted on the proximal end portion 22b of the first driving rod 22 through the motor connection rod 24. This causes the relay holder 42 to tilt, retracting the distal end portion 22a of the first driving rod 22 and advancing the distal end portion 23a of the second driving rod 23. As a result, a driving force is exerted on the ring member 26 of the imaging holder 11, and accordingly, the imaging unit 12 is rotated around the X axis against the urging force of the tension spring 25. During the above operation of changing the viewing direction, predetermined tensile forces are applied between the intermediate portion 23c and the proximal end portion 23b of the second driving rod 23 and between the intermediate portion 22c and the proximal end portion 22b of the first driving rod 22.

The foregoing configuration allows the tensile forces to be exerted at all times on the first and second driving rods 22 and 23 and prevents a force in a buckling direction from being exerted thereon, thus enhancing the reliability of the driving force transmission mechanisms 21. Furthermore, the configuration enables the relay holder 42 to be tilted smoothly.

An exemplary operation in which the motor connection rod 24 is moved backward has been described above. However, the motor connection rod 24 may be moved forward. In this case also, tensile forces similar to the above are exerted on the first and second driving rods 22 and 23 by the tensile spring 25. Furthermore, though only the operation around the X axis has been illustrated in the foregoing, the operation may be performed around the Y axis in a similar manner. In addition, the electric motors 55X and 55Y may be operated concurrently or sequentially to rotate the imaging unit 12 around the two axes. For instance, by changing the driving speed of each of the electric motors 55X and 55Y in a sinusoidal shape and controlling the phase thereof, it is possible to cause the center of the image capturing range to move on an arc or on what is generally-called a Lissajous figure.

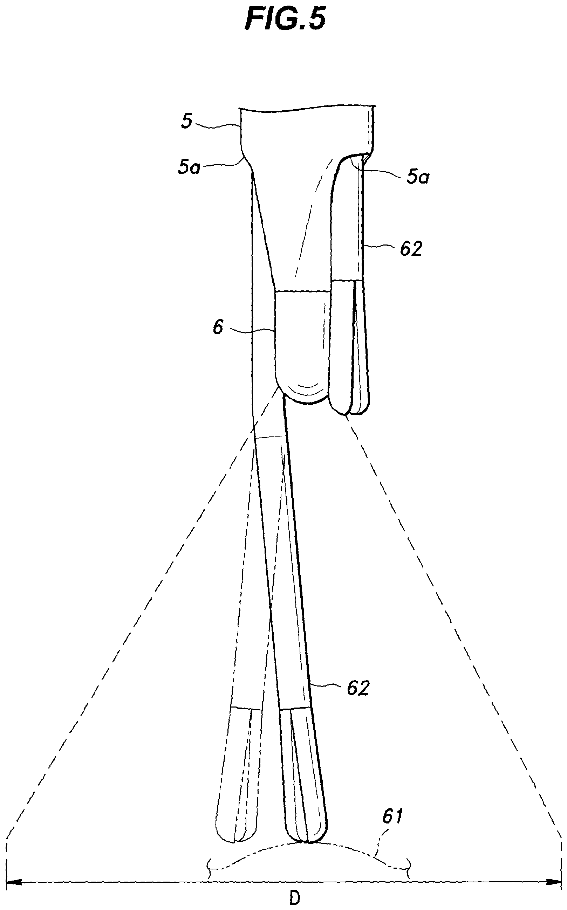

FIG. 5 is a diagram showing an image capturing range and an accessing state of a treatment tool. The imaging unit 12 of the endoscope device 1 according to the present invention can undergo pan/tilt movement with respect to the center axis C when capturing an image, and an effective image capturing range thereof may be, for example, a predetermined range (inside a circle having a diameter D) around an affected part 61, which is an object to be imaged, as shown in FIG. 5. The mechanism for enabling such movement of the imaging unit 12 is accommodated in the aforementioned tubular portion 4a (refer to FIG. 2).

Next, description will be given of the guide portions 4b through which treatment tools are passed. As shown in FIGS. 2 and 3, the guide portions 4b are disposed on respective sides of the tubular portion 4a, and extend in parallel with the tubular portion 4a. The guide portions 4b is configured such that base end portions thereof extend into the main body 2 and distal end portions thereof are connected with front openings 5a via side portions of the intermediate cover 5. The part of the intermediate cover 5 connected with the protection rod 4 has a cross section identical with that shown in FIG. 2. Further, the intermediate cover 5 is configured such that its cross-sectional shape changes from an elliptical shape at an intermediate part thereof to a circular shape at a distal end thereof, so that the part of the intermediate cover 5 connected with the distal end cover 6 has a circular cross section identical with the circular cross section of the distal end cover 6. The openings 5a are defined at the part of the intermediate cover 5 where the shape of the cross section starts changing from the elliptical shape to the circular shape, and an edge of each opening 5a on the side of the center axis C is positioned so as to be in contact with an outer peripheral surface of the distal end cover 6.

As shown in FIGS. 1 and 2, a treatment tool such as a forceps 62 is passed through each guide portion 4b. The forceps 62 is well known and description of the way of handling and operating the forceps 62 will be omitted. It is to be noted, however, that, as shown in FIG. 2, a lengthwise intermediate portion of the forceps 62 is constituted of a fixed shaft 62a in the shape of a circular pipe, a rotation shaft 62b received in the fixed pipe 62a in a double-pipe structure so as to be rotatable in the circumferential direction, and links 62c (two in the drawing) received in the rotation shaft 62b so as to be axially displaceable. In the case where such a forceps 62 is used, each guide portion 4b is formed as a through hole having a sufficient inner diameter to receive the fixed shaft 62a therethrough.

In the endoscope device 1 configured as described above, the linking mechanism enables capturing of an image of any part within the predetermined circular range D, as shown in FIG. 5, and a user can operate the forceps 62 while viewing the captured image displayed on a monitor (not shown in the drawings) connected with the main body 2. Further, as shown in FIG. 5, the forceps 62 is configured to have a curved distal end portion, and thus, rotation of the rotation shaft 62b of the forceps 62 can change the position of the distal end of the forceps 62, as shown by a long dashed double-short dashed line in FIG. 5. The imaging unit 12 and the forceps 62 are provided in the single insertion portion 3 integrally, and the part to be imaged and the position of the forceps 62 can be arbitrarily changed without rotating the insertion portion 3, and thus, it is prevented that the hole for insertion of the insertion portion 3 is widened unnecessarily.

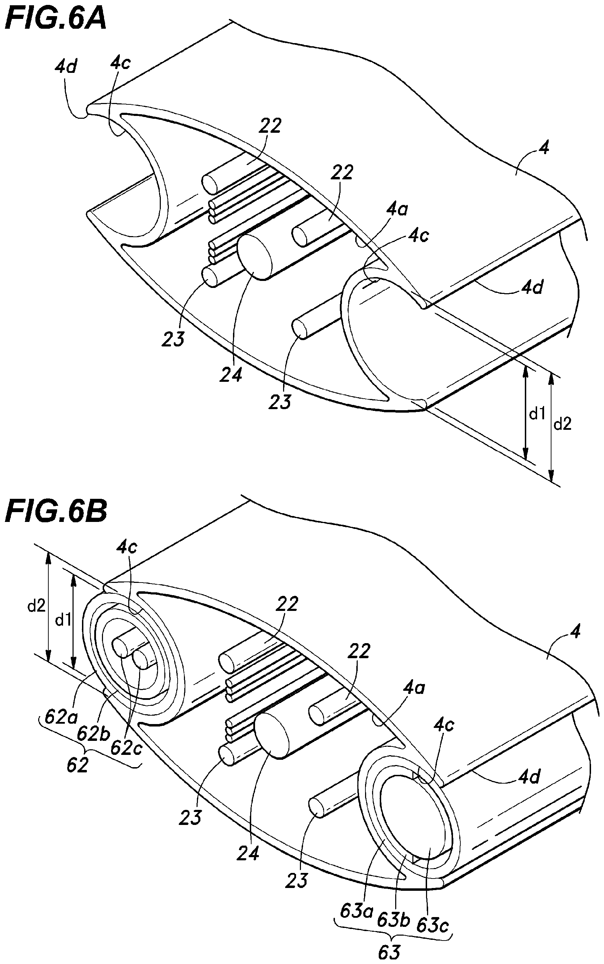

FIG. 6A is a diagram showing a state where the treatment tool has not been mounted in another embodiment of the present invention, and FIG. 6B is a diagram showing a state where the treatment tool has been mounted in the embodiment. In the foregoing embodiment (refer to FIG. 2), each guide portion 4b is formed of a hole having a closed circumference with a circular cross section. However, as shown in FIG. 6A, it may be realized as a guide groove 4c opening on a lateral side of the insertion portion 3 (protection tube 4). In this case, the guide groove 4c is configured such that the opening portion 4d thereof has a width d2 smaller than an inner diameter d1 of the guide groove 4c that is adjusted to correspond to an outer diameter of the fixed shaft 62a of the forceps 62 (d1 > d2). Thereby, as shown in FIG. 6B, the forceps 62 fitted in the guide groove 4c is prevented from inadvertently moving out of the guide groove 4c. The provision of the guide groove 4c opening on the lateral side eliminates the wall thickness on the lateral side and reduces the width (length of the major axis of the ellipse) of the insertion portion 3 (protection tube 4), and this can result in a smaller hole cut in the patient's body for insertion of the insertion portion 3, and thus, can make the surgery scars less conspicuous.

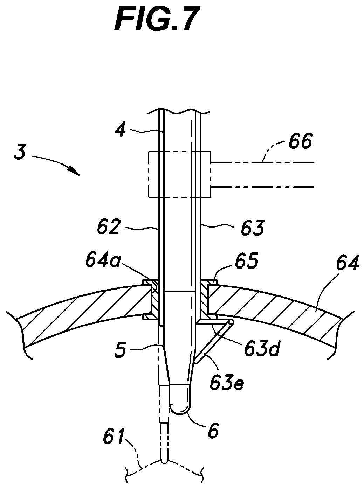

It is to be noted that, in the example shown in FIG. 6B, a forceps 62 is disposed in one of the guide grooves 43 as in the foregoing embodiment and a tensile stretching tool 63 is disposed in the other one of the guide grooves 4c. The part of this tensile stretching tool 63 held in the guide groove 4c includes a fixed shaft 63a identical with that of the forceps 62, a drawing shaft 63b received in the fixed shaft 63a in a double-pipe structure so as to be displaceable in the axial direction and having a C-shaped cross section, and a guide shaft 63c that guides the inner periphery of the drawing shaft 63b in the axial direction. Further, as shown in FIG. 7, a distal end portion of the tensile stretching tool 63 includes a first link 63d having one end pivotably connected with the fixed shaft 63a and a second link 63e having ends pivotably connected with the first link 63d and the drawing shaft 63b, respectively.

FIG. 7 is a diagram showing an exemplary state of use of the endoscope device 1 having a structure as shown in FIG. 1 to FIG. 6. An abdominal wall 64 of a patient is cut open to make a hole 64a of a prescribed size, a trocar 65 is inserted into the hole 64a as required, and the insertion portion 3 is inserted inside the body via the trocar 65. If the major axis of the elliptical shape of the insertion portion 3 is aligned with the direction of cutting, the widening of the hole 64a can be minimized. It is also preferred if the trocar 65 have an elliptical cross section. Then, in a state where the insertion portion 3 has been inserted to a predetermined position, the tensile stretching tool 63 is operated to make the links 63d and 63e protrude sideways so as to abut the trocar 65, and thereafter, the endoscope device 1 is displaced in the drawing-out direction by a prescribed amount and the protection tube 4 is fixed by use of a stand 66, for example.

Thereby, a sufficient working space can be created in the abdominal wall 64. Thereafter, a prescribed treatment can be readily performed by operating the forceps 62 to access the affected part 61, for example.

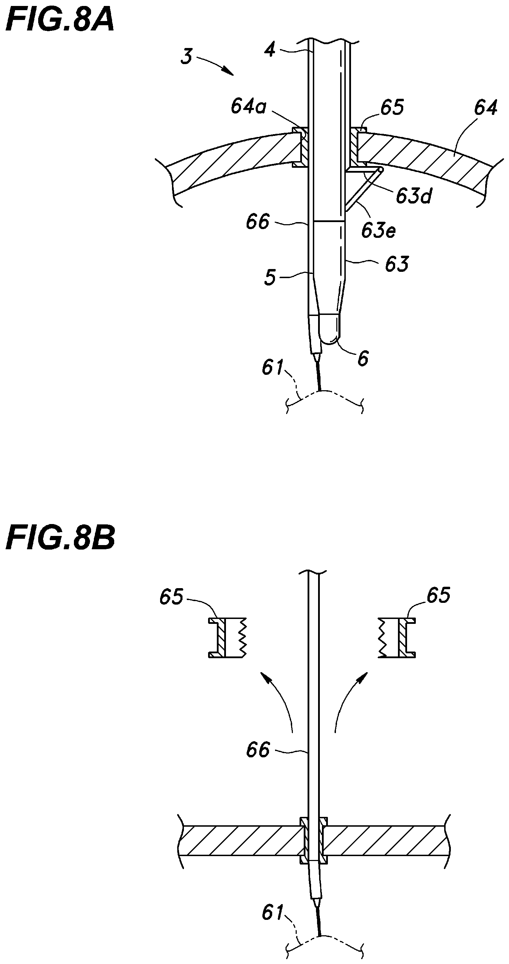

FIG. 8 is a diagram showing another state of use of the endoscope device 1 according to the present invention. In FIG. 8, the parts similar to those in FIG. 7 are denoted by same reference numerals, and detailed description thereof is omitted.

In an example shown in FIG. 8A, an intravenous drip tube 66 is provided in place of the forceps 62 in the example shown in FIG. 7. As in the foregoing embodiment, in a state where the abdominal wall 64 is lifted up to secure a field of view, a needle of the intravenous drip tube 66 is inserted into the affected part 61. Thereafter, the insertion portion 3 is drawn out, while the needle of the drip tube 66 is left inserted. Further, the trocar 65 is cut separate and removed. If necessary, a fixing member 67 may be fitted into the hole 64a to hold the drip tube 66.

In the above-illustrated embodiments, the insertion portion 3 is provided with a pair of guide holes 4b or a pair of guide grooves 4c on either side thereof, but it is possible that the insertion portion 3 may have a guide hole 4b on one side and a guide groove 4c on the other. In any case, the imaging unit 12 and the treatment tool (62, 63, 66) can be treated as a unit, and this makes it easier to access the imaged part. Further, the imaging unit 12 with a pan/tilt mechanism provides a wide effective image capturing range D, namely, makes it possible to capture an image over a wide range while keeping the insertion portion 3 immobile, and this improves the ease of handling of the endoscope with the treatment tool (62, 63, 66) integrally mounted thereto.

### INDUSTRIAL APPLICABILITY

In the endoscope device according to the present invention, it is only necessary to insert the endoscope device through a single hole to enable image capture to be performed and to permit the treatment tool to access the affected part, and thus, the endoscope device is useful in the field where an endoscope is used.

### GLOASSARY

- 1: endoscope device
- 3: insertion portion
- 4: protection tube
- 4a: tubular portion
- 4b: guide hole
- 4c: guide groove
- 5: intermediate cover
- 5a: opening
- 12: imaging unit
- 62: forceps
- 63: tensile stretching tool
- 66: intravenous drop tube

## Claims

1. An endoscope device (1) having a main body (2) and an insertion member (3) extending from the main body, the insertion member (3) having high rigidity and being suitable to be inserted into an object through a hole cut open in the object, the endoscope device (2) comprising:
an imaging unit (12) arranged at a distal end of the insertion member and movably supported by the insertion member (3) opposite the main body (1) for capturing an image of the object;
an operation member (22, 23) received in a tubular portion (40) of the insertion member (3) coaxially with the imaging unit (12), the operation member (22, 23) being configured to set an angle of an image capturing direction of the imaging unit (12) relative to an axial direction of the insertion member (3); and **characterized by** two guide portions (4b, 4c) formed integrally with the tubular portion (4a) to guide a treatment tool (62, 63) for accessing the object such that the treatment tool (62, 63) is displaceable in a longitudinal direction of the tubular portion (4a) on a lateral side of the tubular portion (4a), wherein the two guide portions radially interpose the tubular portion (4b, 4c) therebetween.

2. The endoscope device according to claim 1, wherein the at least one of guide portions (4c) is formed of a groove having an opening of a size determined so as to be capable of preventing the treatment tool (62, 63) from coming off from the groove (4a) sideways.

3. The endoscope device (1) according to any one of claims 1 and 2, wherein a part of the insertion member (3) where the guide portions (4b, 4c) are provided has an elliptical cross section.

4. The endoscope device (1) according to any one of claims 1 to 3, wherein the operation member (22, 23) comprises two pairs of rods (22, 23), connected to an imaging holder at mutually diagonal positions and extending in the front-back direction.

5. The endoscope device (1) according to any one of claims 1 to 4, wherein the treatment tool (62) is a pair of forceps.

## Patentansprüche

1. Endoskopvorrichtung (1) mit einem Hauptkörper (2) und einem Einführelement (3), das sich von dem Hauptkörper erstreckt, wobei das Einführelement (3) eine hohe Steifigkeit aufweist und dazu geeignet ist, durch ein Loch in ein Objekt eingeführt zu werden, das in dem Objekt aufgeschnitten ist, wobei die Endoskopvorrichtung (1) aufweist:
eine Bildaufnahmeeinheit (12) zum Aufnehmen eines Bildes des Objektes, die an einem distalen Ende des Einführelements angeordnet ist und durch das Einführelement (3) gegenüberliegend zu dem Hauptkörper (2) beweglich getragen wird,
einem Bedienungselement (22, 23), das in einem röhrenförmigen Abschnitt (40) des Einführelements (3) koaxial mit der Bildaufnahmeeinheit (12) aufgenommen ist, wobei das Bedienungselement (22, 23) ausgestaltet ist, einen Winkel einer Bildaufnahmerichtung der Bildaufnahmeeinheit (12) relativ zu einer axialen Richtung des Einführelements (3) einzustellen, und **gekennzeichnet ist durch**
zwei Führungsabschnitte (4b, 4c), die integral mit dem röhrenförmigen Abschnitt (4a) gebildet sind, um ein Behandlungswerkzeug (62, 63) zum Erreichen des Objekts zu führen, derart, dass das Behandlungswerkzeug (62, 63) in einer Längsrichtung des röhrenförmigen Abschnitts (4a) auf einer lateralen Seite des röhrenförmigen Abschnitts (4a) versetzbar ist, wobei die zwei Führungsabschnitte (4b, 4c) den röhrenförmigen Abschnitt radial zwischen sich einschließen.

2. Endoskopvorrichtung nach Anspruch 1, wobei der wenigstens eine der Führungsabschnitte (4c) aus einer Rinne gebildet ist, die eine Öffnung einer Größe aufweist, die so bestimmt ist, dass sie geeignet ist, zu verhindern, dass das Behandlungswerkzeug (62, 63) aus der Rille (4a) zur Seite freikommt.

3. Endoskopvorrichtung (1) nach einem der Ansprüche 1 und 2, wobei ein Teil des Einführelements (3), an dem die Führungsabschnitte (4b, 4c) vorgesehen sind, einen elliptischen Querschnitt aufweist.

4. Endoskopvorrichtung (1) nach einem der Ansprüche 1 bis 3, wobei das Bedienungselement (22, 23) zwei Paare von Stäben (22, 23) aufweist, die mit einem Bildaufnahmehalter an zueinander diagonalen Positionen verbunden sind und sich in der Vorwärts-Rückwärts-Richtung erstrecken.

5. Endoskopvorrichtung (1) nach einem der Ansprüche 1 bis 4, wobei das Behandlungswerkzeug (62) ein Zangenpaar ist.

## Revendications

1. Dispositif d'endoscope (1) comprenant un corps principal (2) et un élément d'introduction (3) qui s'étend depuis ledit corps principal, dans lequel l'élément d'introduction (3) présente une rigidité importante et est apte à être introduit dans un objet à travers un trou coupé dans l'objet, le dispositif d'endoscope (1) comprenant:
une unité de prise de vue (12) destinée à capturer une image de l'objet, qui est disposée à une extrémité distale de l'élément d'introduction et est supportée de manière mobile par l'élément d'introduction (3) en vis-à-vis du corps principal (2),
un élément de manoeuvre (22, 23) qui est reçu de façon coaxiale avec l'unité de prise de vue (12) à l'intérieur d'une portion tubulaire (40) de l'élément d'introduction (3), dans lequel ledit élément de manoeuvre (22, 23) est configuré pour régler un angle d'une direction de capture d'image de ladite unité de prise de vue (12) par rapport à une direction axiale de l'élément d'introduction (3), et est **caractérisé par**
deux portions de guidage (4b, 4c) qui sont formées intégralement avec la portion tubulaire (4a) pour guider un outil de traitement (62, 63) pour accéder à l'objet, de telle sorte que l'outil de traitement (62, 63) est déplaçable dans une direction longitudinale de la portion tubulaire (4a) sur un côté latéral de la portion tubulaire (4a), dans lequel les deux portions de guidage (4b, 4c) enfermant radialement entre elles ladite portion tubulaire.

2. Dispositif d'endoscope selon la revendication 1, dans lequel ladite au moins une des portions de guidage (4c) est formée d'une rainure ayant une ouverture d'une taille déterminée de manière à être apte à empêcher l'outil de traitement (62, 63) de sortir latéralement de la rainure (4a).

3. Dispositif d'endoscope (1) selon l'une quelconque des revendications 1 et 2, dans lequel une partie de l'élément d'introduction (3) sur laquelle les portions de guidage (4b, 4c) sont prévues présente une section transversale elliptique.

4. Dispositif d'endoscope (1) selon l'une quelconque des revendications 1 à 3, dans lequel ledit élément de manoeuvre (22, 23) comprend deux paires de tiges (22, 23) qui sont reliées à un support de prise de vue sur des positions diagonales l'une par rapport à l'autre et s'étendent dans la direction avant-arrière.

5. Dispositif d'endoscope (1) selon l'une quelconque des revendications 1 à 4, dans lequel ledit outil de traitement (62) est une paire de pinces.
